# EUROPEAN PATENT APPLICATION

(11) **EP 4 368 635 A1**
(43) Date of publication of application: **15.05.2024**
(21) Application number: 22838005.1
(22) Date of filing: 07.07.2022
(51) Int. Cl.: C07K 14/33, C12N 9/64, C07K 1/20, C07K 1/18

(54) **METHOD FOR PURIFICATION OF CLOSTRIDIUM BOTULINUM NEUROTOXIN PROTEIN DEPRIVED OF NON-TOXIN PROTEIN**

(30) Priority: 08.07.2021 KR 20210089883
(71) Applicant: Pharma Research Bio Co., Ltd., Gangneung-si, Gangwon-do 25451 (KR)
(72) Inventor: BAEK, Seung Gul, Seongnam-si, Gyeonggi-do 13486 (KR); KIM, Hyung Gun, Seongnam-si, Gyeonggi-do 13486 (KR); KIM, Jeong Han, Seongnam-si, Gyeonggi-do 13486 (KR)
(74) Representative: Cabinet Beau de Loménie
(86) International application number: PCT/KR2022/009828
(87) International publication number: WO 2023/282653

(57) **Abstract**

The present invention relates to a method of purifying a non-toxic protein-removed *Clostridium botulinum* neurotoxin protein.

## Description

### [Technical Field]

The present invention relates to a method of purifying a non-toxic protein-removed *Clostridium botulinum* neurotoxin protein.

### [Background Art]

Botulinum toxin is a type of neurotoxic protein produced by bacteria such as *Clostridium botulinum,* and binds irreversibly to the presynaptic nerve terminals, which results in the inhibition of acetylcholine release in synapses, thereby blocking muscle contraction and exhibiting the secondary effect of muscle relaxation. Due to these functions, botulinum toxin has been used for therapeutic and cosmetic purposes since approval by the US FDA in 1989 (KR 10-2010-0107475 A, KR 10-2008-0049152 A, etc.).

Botulinum toxin is used for therapeutic purposes in neuromuscular diseases such as strabismus, torticollis, or blepharospasm, and for cosmetic purposes in removing wrinkles or frown lines, and treating square jaw, hyperhidrosis, or migraine as an injectable formulation. As side effects, cases such as dysphagia, voice change, dry mouth, blurred vision, etc. have been reported, but there have been no direct reports of deaths caused by botulinum toxin yet. Therefore, botulinum toxin is evaluated as a very safe drug when used appropriately.

In particular, pure neurotoxin protein excluding non-toxic proteins from botulinum toxin may be useful for indications that require repeated administration, and accordingly, there is a need for an improved purification method of isolating a stable and biologically active botulinum neurotoxin protein.

### [Disclosure]

### [Technical Problem]

The present inventors intend to provide an improved purification method of isolating a stable and biologically active botulinum neurotoxin protein.

### [Technical Solution]

An object of the present invention is to provide a method of purifying a non-toxic protein-removed *Clostridium botulinum* neurotoxin protein.

### [Advantageous Effects]

By using a purification method of the present invention, a highly pure botulinum neurotoxin protein may be obtained economically and efficiently.

### [Brief Description of the Drawing]

FIG. 1 shows the results of SDS-PAGE, i.e., sodium dodecyl sulfate polyacrylamide gel electrophoresis of a purified 150 kD-botulinum neurotoxin.
FIG. 2 shows the results of HPLC, i.e., size exclusion chromatography (SEC-HPLC) of the purified 150 kD-botulinum neurotoxin.
FIG. 3 shows the results of examining purity of the obtained sample by electrophoresis when a DEAE column containing tertiary amine or a Q column containing quaternary ammonium is used in the step of performing anion chromatography (HC: heavy chain, LC: light chain).

### [Detailed Description of Preferred Embodiments]

The present invention will be described in detail as follows. Meanwhile, each description and embodiment disclosed in this disclosure may also be applied to other descriptions and embodiments. That is, all combinations of various elements disclosed in this disclosure fall within the scope of the present invention. Further, the scope of the present invention is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present invention.

An aspect of the present invention provides a method of purifying a *Clostridium botulinum* neurotoxin protein.

Specifically, the purification method may include the steps of:
(a) loading, on a hydrophobic interaction column, a solution including the *Clostridium botulinum* toxin protein isolated from a culture to capture the toxin and to pass impurities;
(b) isolating the toxin captured in the step (a) to obtain an eluent including the toxin;
(c) obtaining a non-complex neurotoxin protein from the toxin protein which is included in the eluent obtained in the step (b);
(d) loading, on an anion exchange resin column packed with a resin containing tertiary amine, the solution including the non-complex neurotoxin protein obtained in the step (c); and
(e) isolating the non-complex neurotoxin protein captured in the step (d).

In the present invention, there is no time interval between the steps such as "(a), (b), (c), (d)...", or the steps may be carried out simultaneously, or the steps may be performed at any time intervals of seconds, minutes, hours, etc.

As used herein, the term *"Clostridium botulinum* toxin" is, also referred to as "botulinum toxin", a type of proteins derived from *Clostridium botulinum,* and refers to a protein that binds irreversibly to the presynaptic nerve terminals, which results in the inhibition of acetylcholine release in synapses, thereby blocking muscle contraction and exhibiting the secondary effect of muscle relaxation.

Botulinum toxin protein is divided into 7 types from A to G according to serological characteristics. Botulinum toxin type A is the most lethal natural substance known to humans, and in addition to serotype A, other six serotypes of botulinum toxins which are generally immunologically distinct have been identified, i.e., botulinum toxin serotypes B, C, D, E, F, and G. Different serotypes may be identified by neutralization with type-specific antibodies. The different serotypes of botulinum toxin vary in the severity of the paralysis they evoke and in the animal species that they affect.

The molecular weight of the botulinum toxin protein molecule, for all seven of the known botulinum toxin serotypes, is about 150 kD. Meanwhile, the botulinum toxins are released by *Clostridial bacterium* as complexes including the 150 kD botulinum toxin protein molecule along with associated non-toxin proteins. Thus, the botulinum toxin type A complex may be produced by *Clostridial bacterium* as 900 kD, 500 kD and 300 kD forms. Botulinum toxin types B and C may be produced as 500 kD complexes, and botulinum toxin type D may be produced as 300 kD and 500 kD complexes. Botulinum toxin types E and F may be produced as 300 kD complexes. These complexes (i.e., molecular weight greater than about 150 kD) are believed to include a non-toxin hemagglutinin protein and a non-toxin and non-toxic non-hemagglutinin protein.

The botulinum toxin protein includes a high-molecular-weight complex form containing a pure neurotoxic component of about 150 kD and non-toxin proteins. Thus, the complexed form may include the botulinum neurotoxin protein and one or more non-toxic hemagglutinin proteins, and/or one or more non-toxic non-hemagglutinin protein. Specifically, the botulinum toxin complex protein may be a complex of botulinum neurotoxin (BoNT), non-toxic non-hemagglutinin (NTNHA), and hemagglutinin(HA) proteins. The molecular weight of the complex may be higher than about 150 kD. For example, the complexed form of the botulinum toxin type A may have a molecular weight of about 900 kD, about 500 kD, or about 300 kD.

In one embodiment, the botulinum toxin of the present invention may be type A toxin.

The botulinum neurotoxin (BoNT) protein of the present invention refers to, unless otherwise indicated, a botulinum neurotoxin protein that is not combined with any combined non-toxic proteins such as non-toxic non-hemagglutinin, hemagglutinin, etc., and it may also be referred to as "non-complex botulinum neurotoxin protein", "pure neurotoxin protein" or "non-toxic protein-removed (reduced) botulinum neurotoxin protein".

In one embodiment, the botulinum neurotoxin protein of the present invention may be a protein having a molecular weight of about 50 kD to about 150 kD. Specifically, the botulinum neurotoxin protein of the present invention includes all of about 100 kD heavy chain (HC) conjugated to about 50 kD light chain (LC) via a disulfide bond, or about 150 kD single-chain precursor protein of the neurotoxin component.

As used herein, the term "about" includes the exact number following the term as well as approximately the number or ranges near to the number. Considering the context in which the number is presented, whether a number is near to or approximately a specifically recited number may be determined. For example, the term "about" may refer to a range of -10% to +10% of a numeric value. For another example, the term "about" may refer to a range of -5% to +5% of a given numerical value. However, it is not limited thereto.

In any one embodiment of the above-described embodiments, the *Clostridium botulinum* toxin protein of the present invention may be obtained from a culture of *Clostridium botulinum.* Specifically, the protein may be isolated from the culture of *Clostridium botulinum.*

Therefore, the purification method of the present invention may further include the step of culturing *Clostridium botulinum,* prior to the chromatography step for purification, but is not limited thereto.

In the culturing step, the culturing of the *Clostridium botulinum* strain may be performed according to an appropriate medium and culture conditions known in the art. Specifically, the *Clostridium botulinum* strain may be cultured in a common medium containing appropriate carbon sources, nitrogen sources, phosphorus sources, inorganic compounds, etc. under anaerobic conditions while controlling temperature, pH, etc.

For example, the culturing may be performed under anaerobic conditions at about 25°C to 40°C, specifically, at 27°C to 40°C, but is not limited thereto. The culturing period may continue until a desired production amount of the toxin protein is obtained, specifically, for about 12 hours to 150 hours, but is not limited thereto.

In any one embodiment of the above-described embodiments, the solution including the *Clostridium botulinum* toxin protein of the present invention may be subjected to precipitation treatment prior to the chromatography step.

In one embodiment, the precipitation may be acid precipitation, but is not limited thereto. For example, the acid precipitation may include adding an acid to pH 3.0 to pH 4.0, specifically pH 3.3 to 3.5, or more specifically pH 3.4 to 3.5. In the acid precipitation, an acidic solution known in the art, for example, sulfuric acid or hydrochloric acid, etc. may be used, but is not limited thereto.

In any one embodiment of the above-described embodiments, the solution including the *Clostridium botulinum* toxin complex protein of the present invention may be subjected to filtration treatment prior to the hydrophobic chromatography step. Specifically, the solution may be subjected to filtration treatment after the acid precipitation.

The filtration step may be performed by a known process such as microfiltration, ultrafiltration, precision filtration, and depth filtration, and impurities may be removed in the filtration step.

In one embodiment, a device used in the filtration treatment may include a filter having a pore size of about 0.1 µm to about 0.3 µm, specifically, about 0.2 µm, but is not limited thereto.

In the purification method of the present invention, the steps of (a) loading, on a hydrophobic interaction column, the solution including the *Clostridium botulinum* toxin protein to capture the toxin and to pass impurities; and (b) isolating the toxin captured in the step (a) to obtain an eluent including the toxin may be referred to as the step of performing hydrophobic interaction chromatography using the solution including the *Clostridium botulinum* toxin protein, but is not limited thereto.

As used herein, the "chromatography" refers to any process of separating components of a mixture by passing the mixture through a medium such that the components of the mixture pass through the medium at different rates.

The chromatography of the present invention includes column chromatography, planar chromatography, thin layer chromatography, gas chromatography, liquid chromatography, fast protein liquid chromatography (FPLC), and high performance liquid chromatography (HPLC). In each step of the purification process of the present invention, an exemplary type of chromatography process or device is disclosed, which may be applied to all types of chromatography described above.

As used herein, the "hydrophobic chromatography" or "hydrophobic interaction chromatography" is a method of separating molecules based on the relative strength of hydrophobic interaction with a nonpolar stationary phase. The hydrophobic chromatography uses the principle that the higher the salt concentration, the stronger the interaction between a non-polar stationary phase and a substance to be separated, and the lower the ionic strength or salt concentration of a buffer, the weaker the interaction. Therefore, when a descending gradient of the salt concentration is used, less hydrophobic substances may be eluted first, and more hydrophobic substances may be eluted later.

In one embodiment, in the step (a) of the purification method of the present invention, a column having ligands such as ether, isopropyl, butyl, octyl, phenyl, etc. may be used as the hydrophobic interaction column. For example, in the step (a) of the purification method of the present invention, the hydrophobic interaction column may be a butyl sepharose or phenyl sepharose column. Specifically, the hydrophobic interaction column may be a phenyl sepharose column. More specifically, the hydrophobic interaction column may be selected from the group consisting of butyl sepharose high performance (butyl sepharose HP), butyl sepharose fast flow, phenyl sepharose high performance (HP), and phenyl sepharose fast flow columns, but is not limited thereto, and any column may be used without limitation, as long as it belongs to hydrophobic interaction columns.

In any one embodiment of the above-described embodiments, the step of equilibrating the column using a buffer solution may be further included prior to the step (a) of the purification method of the present invention.

As used herein, the equilibration may refer to the step of stabilizing the column with a buffer solution in order to prevent a protein from aggregating or losing activity due to changes in the environment before injecting, into the column, the protein to be purified.

In the equilibration step prior to the step (a), conditions such as a flow rate of flowing the buffer solution, temperature, time, conductivity, etc. may be appropriately adjusted.

For example, a phosphate buffer solution, a citrate buffer solution, or an acetate buffer solution may be used as the buffer solution. For example, the column buffer solution may be a phosphate buffer solution, for example, sodium phosphate. For example, the concentration of the column buffer solution may be adjusted to 5 mM to 100 mM, for example, 25 mM to 75 mM, or 40 mM to 60 mM.

For example, the flow rate may be about 1.0 ml/min to about 20.0 ml/min. For example, the conductivity may be about 170 mS/cm to about 220 mS/cm, but is not limited thereto.

In any one embodiment of the above-described embodiments, the step (a) of the purification method of the present invention may be performed under condition of pH 4 to pH 8, but is not limited thereto.

In any one embodiment of the above-described embodiments, the step (a) of the purification method of the present invention may be performed under conductivity condition of about 170 mS/cm to about 220 mS/cm, but is not limited thereto.

In the step (a) and/or the step (b), the flow rate of flowing the solution, temperature, time, etc. may be appropriately adjusted.

For example, a phosphate buffer solution, a citrate buffer solution, or an acetate buffer solution may be used as the column buffer solution. For example, the column buffer solution may be a phosphate buffer solution, for example, sodium phosphate. For example, the concentration of the column buffer solution may be adjusted to 5 mM to about 100 mM, e.g., 25 mM to 75 mM, or 40 mM to 60 mM. For example, the flow rate of the mobile phase may be about 1.0 ml/min to about 20.0 ml/min, but is not limited thereto.

In any one embodiment of the above-described embodiments, an appropriate elution solvent may be used in the step of obtaining an eluent including the toxin protein in the step (b).

The step (b) may employ a concentration gradient. For example, the toxin complex protein may be eluted using a stepwise salt gradient or continuous salt gradient.

The step (b) may include reducing ionic strength or increasing pH. In the elution step, the hydrophobic moiety of the protein which is adsorbed to the stationary phase may be desorbed to the mobile phase by, for example, starting a reverse salt gradient to reduce the salt concentration. Specifically, in the elution step, a descending concentration gradient of a buffering agent may be used. For example, a buffering agent with a concentration gradient ranging from about 5.0 M to about 0.0 M, about 4.0 M to about 0.0 M, about 3.5 M to about 0.0 M, or about 3.0 M to about 0.0 M may be used. The buffering agent may be, for example, sodium sulfate (Na₂SO₄), sodium chloride (NaCl), potassium chloride (KCI), ammonium acetate (NH₄OAc), etc., and specifically, in the elution step, a concentration gradient of sodium chloride (NaCl) may be used, but is not limited thereto. A buffering agent appropriately selected from the range known in the art may be used.

In any one embodiment of the above-described embodiments, the eluent obtained in the step (b) of the purification method of the present invention may be subjected to precipitation treatment. For example, the precipitation may be acid precipitation, but is not limited thereto.

The acid precipitation may include adding an acid. Specifically, the acid precipitation may be adding ammonium sulfate to the eluent. More specifically, the acid precipitation may be adding ammonium sulfate to the eluent so that the final saturation is 30% to 50%. For example, the concentration of ammonium sulfate at the saturation level may be about 19.6 g/100 ml to about 31.3 g/100 ml, but is not limited thereto.

The precipitated eluent may undergo additional purification processes, for example, filtration and/or centrifugation, etc. The obtained precipitate may be redissolved for additional purification processes. For example, the precipitate may be dissolved using a solution with a pH of 5.0 to 7.0. For example, a sodium phosphate buffer solution with a concentration of about 40 mM to about 60 mM may be used, but is not limited thereto.

In the purification method of the present invention, the step (c) of obtaining a non-complex neurotoxin protein from the toxin protein which is included in the eluent obtained in the step (b) may be dissociating the *Clostridium botulinum* toxin protein into a non-toxic protein and a pure neurotoxin protein.

The step (c) may be performed by any common protein isolation method for separating the pure neurotoxin protein and the non-toxic protein.

In one embodiment, the step (c) may employ a method of extracting a protein from other proteins, such as UF diafiltration, pH titration, dialysis, precipitation, RBC treatment, depth filtration (DF), microfiltration (MF), ultrafiltration (UF), sterile filtration, fraction, HPLC, and/or centrifugation, etc., but any method may be used without limitation as long as it is a method capable of obtaining the pure neurotoxin protein.

In any one embodiment of the above-described embodiments, the step (c) may employ dialysis. The dialysis may be performed by placing the eluent obtained in the step (b) to a membrane and by using a buffer solution under appropriate temperature and/or pH conditions. The number of times of the dialysis may be appropriately adjusted, for example, the dialysis may be performed three times, but is not limited thereto.

For example, the temperature may be about 0°C to about 10°C, specifically, about 2°C to about 8°C. For example, the dialysis may be performed in a buffer solution with a pH of 6 to 10. For example, the buffer solution may be a phosphate buffer solution, a citrate buffer solution, or an acetate buffer solution, and specifically, sodium phosphate may be used. The concentration of the buffer solution may be appropriately adjusted, for example, about 10 mM to 70 mM, but is not limited to the above examples.

In the purification method of the present invention, the step of (d) loading, on an anion exchange resin column, the solution including the non-complex neurotoxin protein obtained in the step (c); and the step of (e) isolating the non-complex neurotoxin protein captured in the step (d) may be referred to as the step of performing anion-exchange chromatography using a solution including the non-complex neurotoxin protein, but is not limited thereto.

As used herein, the term "anion-exchange chromatography" refers to separation of molecules according to their charges by binding negatively charged (or acidic) molecules to a positively charged support, and homologs of the molecules (acidic, basic, and neutral) may be easily separated by this technique.

In the purification method of the present invention, a resin which may be used in the anion-exchange chromatography of the step (d) may be a weak anion exchange resin containing secondary or tertiary amine as a functional group.

In one embodiment, the resin which may be used in the anion-exchange chromatography may be a resin containing tertiary amine. Specifically, the resin may be a resin containing diethylaminoethyl (DEAE), but is not limited thereto. In any one embodiment of the above-described embodiments, the anion exchange resin column of the present invention may be a DEAE Sepharose high performance (DEAE Sepharose HP) or DEAE Sepharose fast flow column, but is not limited thereto.

In any one embodiment of the above-described embodiments, the step of equilibrating the anion exchange resin using a buffer solution may be further included prior to the step (d) of the purification method of the present invention.

In the equilibration step prior to the step (d), conditions such as a flow rate of flowing the buffer solution, temperature, time, conductivity, etc. may be appropriately adjusted.

For example, a phosphate buffer solution, a citrate buffer solution, or an acetate buffer solution may be used as the column buffer solution. Specifically, the column buffer solution may be a phosphate buffer solution, for example, sodium phosphate. For example, the concentration of the column buffer solution may be adjusted to 5 mM to 100 mM, for example, 5 mM to 35 mM, or 10 mM to 30 mM.

For example, the flow rate may be about 0.1 ml/min to about 20.0 ml/min. For example, the conductivity may be about 0.1 mS/cm to 10 mS/cm, but is not limited thereto.

In the step (d) and/or the step (e), the flow rate of flowing the solution, temperature, time, conductivity, etc. may be appropriately adjusted.

For example, a phosphate buffer solution, a citrate buffer solution, or an acetate buffer solution may be used as the buffer solution. For example, the column buffer solution may be a phosphate buffer solution, for example, sodium phosphate. For example, the concentration of the column buffer solution may be adjusted to 5 mM to 100 mM, for example, 5 mM to 35 mM, or 10 mM to 30 mM. For example, the flow rate may be about 0.1 ml/min to about 20.0 ml/min. For example, the conductivity may be about 0.1 to 10 mS/cm, but is not limited thereto.

In any one embodiment of the above-described embodiments, the step (d) of the purification method of the present invention may be performed under condition of pH 4 to pH 10, but is not limited thereto.

In any one embodiment of the above-described embodiments, the step (e) may include changing ionic strength or pH using an appropriate elution solvent.

The step (e) may employ a concentration gradient. For example, elution may be performed using a stepwise salt gradient or continuous salt gradient. Specifically, in the elution step, the proteins bound to the column may be eluted using a buffer solution with an ascending concentration gradient. For example, a buffer with a concentration gradient ranging from about 0.0 M to about 3.0 M, about 0.0 M to about 2.0 M, about 0.0 M to about 1.0 M may be used. In one embodiment, the buffer solution which may be used in the elution step may be sodium chloride (NaCl), potassium chloride (KCI), sodium phosphate, potassium phosphate, or Tris, etc. Specifically, in the elution step, a concentration gradient of sodium chloride (NaCl) may be used, but is not limited thereto, and those appropriately selected within a range known in the art may be used.

In any one embodiment of the above-described embodiments, the purification method of the present invention may not necessarily include the step of loading the solution including the neurotoxin protein onto a cation exchange resin column, but is not limited thereto.

The neurotoxin protein isolated according to the purification method of the present invention may have a high purity. Specifically, the neurotoxin protein may have a purity of about 90% or more, about 95% or more, about 96% or more, about 97% or more, about 97.5% or more, or about 98% or more, but is not limited thereto.

### [Mode for Carrying Out the Invention]

Hereinafter, the present invention will be described in more detail with reference to Examples and Experimental Examples. However, these Examples and Experimental Examples are only for illustrating the present invention, and the scope of the present invention is not intended to be limited by these Examples and Experimental Example.

### Example 1. Culture of botulinum bacteria and Isolation of toxin

*Clostridium botulinum* stored at -80°C was thawed and added to a seed culture medium, and cultured at 37°C under anaerobic conditions for 24 hours to increase the number of bacteria. The culture medium in which the number of bacteria was increased was added to a main culture medium and further cultured at 35°C under anaerobic conditions for 92 hours to 100 hours, and then virus inactivation was performed through acid precipitation to adjust pH to 3.4. When precipitation was completed, the botulinum toxin was eluted with a buffer solution, and then filtered through a 0.2 µm sterile filter without contact with the outside.

### Example 2. Purification of botulinum toxin

### 2-1. Hydrophobic interaction chromatography

The filtered solution containing the botulinum type A toxin of Example 1 was loaded onto a column packed with a phenyl sepharose hydrophobic interaction chromatography resin. Before the loading, equilibration/washing was performed by applying an equilibration/elution buffer solution (50 mM sodium phosphate, 3 M sodium chloride, pH 6.0) at a flow rate of 5 ml/min and a conductivity of 188 mS/cm.

The filtered solution containing the botulinum type A toxin of Example 1 was loaded onto a phenyl sepharose column, and then botulinum toxin was eluted from the column using a descending salt gradient (3.0 M - 0.0 M sodium chloride concentration gradient) of a buffering agent of 50 mM sodium phosphate with a pH of 6.0 under conditions of a flow rate of 5 ml/min and a conductivity of 188 mS/cm.

### 2-2. Ammonium sulfate precipitation and Pure neurotoxin dissociation

Precipitation was performed by adding ammonium sulfate to the eluent collected by the method of Example 2-1 at a final saturation of 40% (24.3 g/100 ml), and then centrifugation was performed to obtain a precipitate. In this process, the toxin protein was also in contact with the inner surface of the sterile container, and there was no contact with the outside. The precipitate was dissolved in a 50 mM sodium phosphate buffer solution (pH 6.0), and then, for dissociation into non-toxic protein and pure neurotoxin protein, the dissolved solution was put in a membrane, followed by dialysis in a 20 mM sodium phosphate buffer solution (pH 7.9) under condition of 2°C to 8°C. The dialysis was performed a total of three times.

### 2-3. Anion exchange resin chromatography

The dialysis solution obtained in Example 2-2 was centrifuged to separate the supernatant, and the separated supernatant was injected into fast protein liquid chromatography (FPLC) connected to a DEAE Sepharose column. As Comparative Example, anion exchange resin chromatography was performed using a Q column instead of the DEAE Sepharose column.

The specific experimental method is as follows. Before injection of the supernatant, the column packed with the DEAE Sepharose resin was equilibrated by applying a sodium phosphate buffer solution (20 mM, pH 7.9) thereto, and the toxin-containing sodium phosphate buffer solution (20 mM, pH 7.9) was applied to the column. After equilibration, the step of injecting the supernatant was performed, and the flow through (FT) fluid was collected in a sterile container while the neurotoxin bound to the anion exchange column material, followed by the washing step, in which a washing buffer solution (20 mM sodium phosphate with a pH of 7.9) was applied through the anion exchange column. The next step was an elution step. The column was washed with a 20 mM sodium phosphate buffer solution with a pH of 7.9, and then the botulinum toxin was eluted from the column using an ascending salt gradient (0.0 M -1.0 M sodium chloride gradient). The anion exchange resin chromatography was performed under conditions of a conductivity of 3 mS/cm and a flow rate of 3 ml/min.

### Example 3. Purity analysis of purified toxin

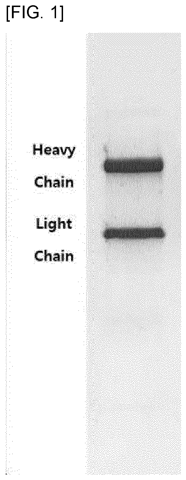

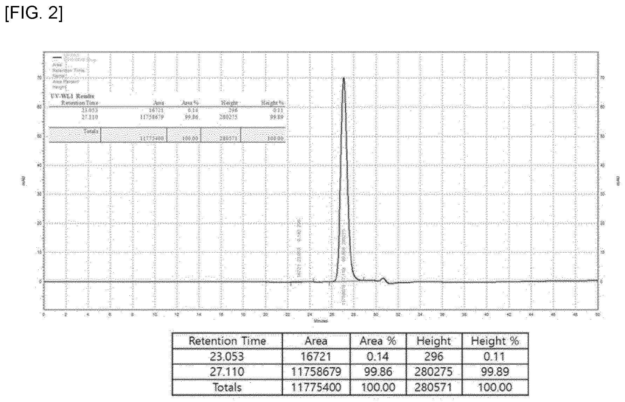

In order to purify the pure neurotoxin from which non-toxic proteins were removed, the sample which was obtained through the purification process from the hydrophobic interaction chromatography to the anion exchange resin chromatography was subjected to a purity analysis through sodium dodecyl sulfate polyacrylamide gel electrophoresis (SDS-PAGE, FIG. 1) and size exclusion chromatography (SEC-HPLC, FIG. 2). Electrophoresis was performed using a 4%-20% polyacrylamide gel, followed by staining with Coomassie blue. As a mobile phase of SEC-HPLC, a 50 mM sodium phosphate solution containing 250 mM NaCl with a pH of 6.0 was used, and an HPLC column was connected, and 20 µg of botulinum type A neurotoxin protein obtained in Example 2 was loaded and applied at a rate of 0.4 mL/mL for 60 minutes.

As a result, no impurity protein bands were observed except for the pure neurotoxin components, and only the pure neurotoxin components of 98 kD (heavy chain) and 52 kD (light chain) were observed (FIG. 1). Further, it was confirmed that the purity of botulinum neurotoxin was 99.86% (FIG. 2).

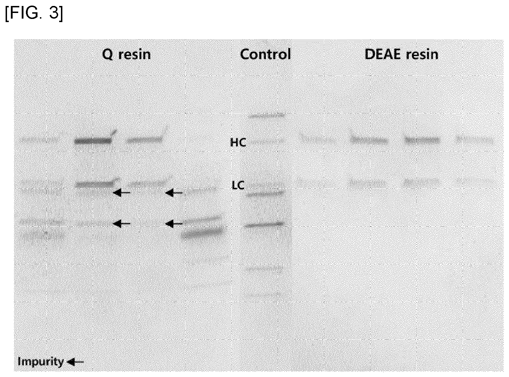

In contrast, as a result of analyzing the purity of the sample, which was obtained using the DEAE column or Q Sepharose column in the anion chromatography step, by electrophoresis (FIG. 3), many impurity protein bands were observed in addition to the pure neurotoxin components, when the Q column containing quaternary ammonium salts was used.

These results indicate that the neurotoxin protein may be purified in higher purity by using the DEAE column containing tertiary amine, as compared to using the Q column containing quaternary ammonium salts.

These results suggest that when the two stages of the hydrophobic interaction chromatography and the anion-exchange chromatography including the DEAE column are performed, the neurotoxin protein from which almost all impurities are removed may be purified with high purity.

Therefore, when the above purification method is used, only the pure neurotoxin portion (about 150 kD) from which the non-toxic proteins of botulinum toxin type A have been removed may be obtained with high purity, indicating that the pure neurotoxin with high efficacy and purity, from which non-toxic proteins have been removed with high efficiency, may be produced using the method and system disclosed in this specification.

Based on the above description, it will be understood by those skilled in the art that the present invention may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A method of purifying a non-complex *Clostridium botulinum* neurotoxin protein, the method comprising the steps of:
(a) loading, on a hydrophobic interaction column, a solution including the *Clostridium botulinum* toxin protein isolated from a culture to capture the toxin and to pass impurities;
(b) isolating the toxin captured in the step (a) to obtain an eluent including the toxin;
(c) obtaining the non-complex neurotoxin protein from the toxin protein which is included in the eluent obtained in the step (b);
(d) loading, on an anion exchange resin column packed with a resin containing tertiary amine, the solution including the non-complex neurotoxin protein obtained in the step (c); and
(e) isolating the non-complex neurotoxin protein captured in the step (d).

2. The method of claim 1, wherein the anion exchange resin column is a DEAE Sepharose HP or DEAE Sepharose fast flow column.

3. The method of claim 1, comprising the step of culturing a *Clostridium botulinum* strain prior to the step (a).

4. The method of claim 3, further comprising the step of performing acid precipitation of the culture of the strain.

5. The method of claim 4, wherein the acid precipitation includes adding an acid to pH 3.0 to pH 4.0.

6. The method of claim 4, further comprising the step of filtering the acid-precipitated solution.

7. The method of claim 1, wherein the hydrophobic interaction column is selected from the group consisting of butyl sepharose high performance (butyl sepharose HP), butyl sepharose fast flow, phenyl sepharose high performance (HP), and phenyl sepharose fast flow columns.

8. The method of claim 1, wherein the step (a) is performed under conditions of pH 4 to pH 8; and conductivity of 170 mS/cm to 220 mS/cm.

9. The method of claim 1, wherein the column buffer solution of the step (a) is a phosphate buffer solution.

10. The method of claim 1, wherein the column buffer solution of the step (b) is a phosphate buffer solution.

11. The method of claim 1, wherein a concentration gradient is used in the step (b).

12. The method of claim 1, further comprising the step of performing acid precipitation of the eluent obtained in the step (b), between the step (b) and the step (c).

13. The method of claim 12, wherein the acid precipitation step includes adding ammonium sulfate to the eluent at a final saturation of 30% to 50%.

14. The method of claim 1, wherein the step (c) is dissociating the *Clostridium botulinum* toxin protein into a non-toxic protein and a pure neurotoxin protein.

15. The method of claim 1, wherein the step (c) includes any one or more selected from the group consisting of UF diafiltration, pH titration, dialysis, and precipitation.

16. The method of claim 1, wherein the step (c) is performing dialysis three times using a sodium phosphate buffer solution.

17. The method of claim 1, wherein the column buffer solution of the step (e) is a phosphate buffer solution.

18. The method of claim 1, wherein a concentration gradient is used in the step (e).

19. The method of claim 1, wherein the *Clostridium botulinum* neurotoxin protein purified by the method has a purity of 98% or more.

20. The method of claim 1, not comprising the step of loading, on a cation exchange resin column, the solution including the neurotoxin protein.

21. The method of claim 1, wherein the non-complex neurotoxin protein has a molecular weight of 50 kD to 150 kD.
